# EUROPEAN PATENT APPLICATION

(11) **EP 3 677 576 A1**
(43) Date of publication of application: **08.07.2020**
(21) Application number: 19150201.2
(22) Date of filing: 03.01.2019
(51) Int. Cl.: C07D 277/74, C07B 45/06

(54) **FLUORINE-CONTAINING COMPOUNDS FOR USE AS NUCLEOPHILIC REAGENTS FOR TRANSFERRING FUNCTIONAL GROUPS ONTO HIGH VALUE ORGANIC COMPOUNDS**

(71) Applicant: Freie Universität Berlin, 14195 Berlin (DE)
(72) Inventor: HOPKINSON, Matthew, 14167 Berlin (DE); DIX, Stefan, 10319 Berlin (DE)
(74) Representative: Maikowski & Ninnemann Patentanwälte Partnerschaft mbB

(57) **Abstract**

The present invention relates to a compound of general formulae (I) 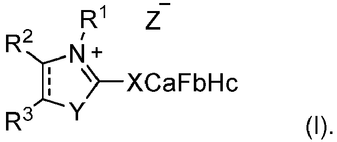 for use as reagents in trifluoromethylthiolation reactions.

## Description

The present invention relates to fluorine-containing compounds for use as nucleophilic reagents for transferring functional groups onto high value organic compounds and methods for synthesizing them.

### Description

Fluoroalkyl groups, in particular fluoroalkyl-containing thiol-groups, such as SCF₃, are attracting increasing attention in medicinal chemistry as a substituent that when incorporated into pharmaceuticals and other biologically-active compounds, can improve their potency and bioavailability.

In particular, the SCF₃ group is one of the most lipophilic moieties available and can allow for an increase in the membrane permeability of drug targets. To date, several SCF₃-containing pharmaceuticals have been introduced and medicinal chemistry programs now routinely investigate SCF₃-substituted derivatives of potential drug candidates alongside those labelled with more traditional fluorinated (eg. trifluoromethyl, CF₃) or non-fluorinated groups (scheme 1).

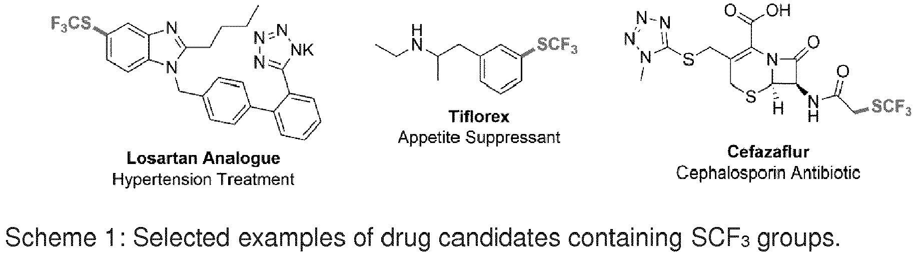

The synthesis of SCF₃-containing molecules can be achieved using a number of different strategies. A useful approach in the context of medicinal chemistry is direct trifluoromethylthiolation wherein the SCF₃ moiety is attached directly as a whole intact group onto a target substrate. These reactions can potentially be conducted on the same substrates used when installing other related groups (eg. CF₃) and thus are simple to incorporate into screening studies.

Until the last few years, there existed only a few known reagents such as the highly toxic and difficult to handle gaseous species SCF₃X (X = Cl, Br) and F₃CSSCF₃ for electrophilic trifluoromethylthiolation reactions.

In recent years, however, a number of bench-stable and easy-to-use electrophilic trifluoromethylthiolating reagents have been developed and, as a result, the use of the SCF₃ group in research laboratories and in pharmaceutical development has increased dramatically (Scheme 2).

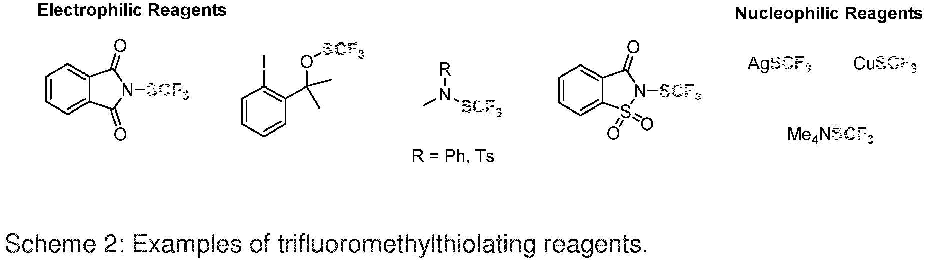

However, nucleophilic sources of SCF₃ are limited to a relatively small number of stable metal or ammonium salts [M][SCF₃] (M = eg. Ag, Cu, Me₄N) which, in most cases, contain rather expensive metal cations.

It is thus desirable to provide alternative nucleophilic sources for fluoroalkyl-containing thiol-groups, which do not require metal cations and are purely organic.

Furthermore, nucleophilic sources of related fluoroalkyl-containing groups such as SCₙF₂ₙ₊₁ (n > 1) or SCF₂H are even more scarce due to the instability of simple metal salts with these anions.

The incorporation of fluoroalkyl selenyl (eg. SeCF₃) or fluoroalkoxy (eg. OCF₃) groups into organic molecules would also benefit from the availability of new practical nucleophilic reagents.

These objectives are solved by compounds as described in claim 1.

Accordingly, a fluorine containing compound of the general formulae (I) 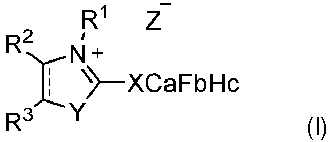 is provided, wherein
- R¹ is C1-C20 alkyl;
- R², R³ are in each case an alkyl, a cycloalkyl, an aryl, a heteroaryl, a halogen, a halogen substituted alkyl or both R² and R³ are part of a cyclic system;
- X is S, O, Se, Te; preferably S, O, Se;
- Y is S, O, NR⁵ where R⁵ is an alkyl or aryl;
- Z⁻ is R⁴SO₃⁻ with R⁴ being H, C1-C10 alkyl, aryl, CaFbHc, in particular ⁻OTf (CF₃SO₃⁻), PhSO₃- or p-Tos-; I⁻, Cl⁻, ClO₄⁻, BF₄⁻;
- a is 1-20, preferably 1-12, more preferably 1-8;
- b is (2a+1) - c,
- c is 0-10, preferably 0-5, more preferably 0, 1, 2.

Rather than acting as an electrophilic trifluoromethylthiolating reagent, the compound of formulae (I) acts a source of nucleophilic ⁻XCₐF_{b}H_{c}, such as ⁻SCF₃. Unlike most existing species, this compound does not contain expensive metal cations and is purely organic in nature. The compound of general formulae (I) is a solid that is easy to handle under ambient conditions and is bench-stable over extended periods.

In an embodiment of the present compound moiety R¹ is C1-C10 alkyl, preferably C1-C5 alkyl, more preferably C1-C3 alkyl. In a particular preferred embodiment moiety R¹ is a methyl group (-CH₃).

In a further embodiment of the present compound the moieties R² and R³ are part of an aromatic system, preferably of a C6-C10 aryl ring, more preferably of a C6 aryl ring, which may be further substituted. This may comprise a non-substituted or substituted C6 aryl ring or non-substituted or substituted naphthyl ring.

The moiety R⁵ may be a C1-C10 alkyl, preferably C1-C5 alkyl, more preferably C1-C3 alkyl. In a particular preferred embodiment moiety R⁵ may be a methyl, ethyl, isopropyl etc. The moiety R⁵ may be also a C6-C10 aryl ring, more preferably of a C6 aryl ring, for example a phenyl, 2,4,6-trimethylphenyl or 2,6-diisopropylphenyl.

It is to be understood that the moieties R¹, R², R³ and R⁵ can be non-substituted or further substituted.

Here the term "substituted", in particular in connection to alkyl, cycloalkyl, aryl or heteroaryl relates to the substitution of one or more atoms, usually H-atoms, by one or more of the following substituents: halogen, hydroxy, protected hydroxy, oxo, C₃-C₁₀-cycloalkyl, aryl, heteroaryl, naphthyl, imino, imido, isocyano, amino, protected amino, primary or secondary amino, heterocyclic ring, carbonate, imidazolyl, indolyl, pyrrolidinyl, C₁-C₁₂-alkoxy, C₁-C₁₂-acyl, C₁-C₁₂-acyloxy, nitro, nitroso, carboxy, ester, aldehyde, ketone, sulfonic acid, sulfinic acid, thiocarbonyl, phosphate, phosphonate, boronate, carbamoyl, carboxamide, N-(C₁-C₁₂-alkyl)carboxamide, N,N-Di(C₁-C₁₂-alkyl)carboxamide, cyano, alkylsulfonylamino, arylsulfonylamino, arylsulfonyl, alkylsulfinyl, arylsulfinyl, thiol, C₁-C₁₀-alkylthio, arylthiol, C₁-C₁₂-(per)fluoroalkyl and C₁-C₁₀-alkylsulfonyl. The substituted groups can be once or twice substituted with same or different substituents. The alkyl moieties may also comprise one or multiple double bonds.

In another embodiment the present compound is of the general formulae (II) 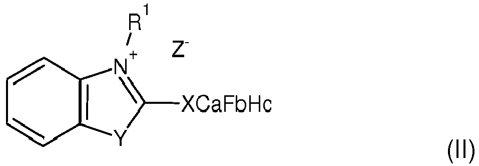 wherein R¹, X, Y, Z, a, b, c have the above meanings.

In yet a further embodiment the present compound is of the general formulae (III) 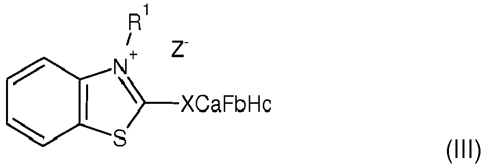 wherein R¹, X, Z, a, b, c have the above meanings.

In still another embodiment the present compound is of the general formulae (IV) 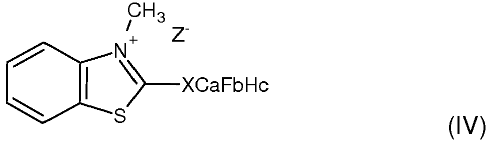 wherein X, Z, a, b, c have the above meanings.

In a variant wherein c = 0 the moiety -XCₐF_{b}H_{c} does not contain any hydrogen. In such a case the moiety is -XCaFb, as for example -XCF₃, -XCF(CF₃)₂ or -XC₈F₁₇, in particular -SCF₃, -SeCF₃, -OCF₃ or -SC₈F₁₇. However, any perfluoralkyl moiety is suitable.

In variants wherein c = 1 or 2 the moiety -XCₐF_{b}H_{c} may be -XCF₂H or -XCFH₂, in particular -SCF₂H or -SCFH₂.

Preferred embodiments of the present compound include: 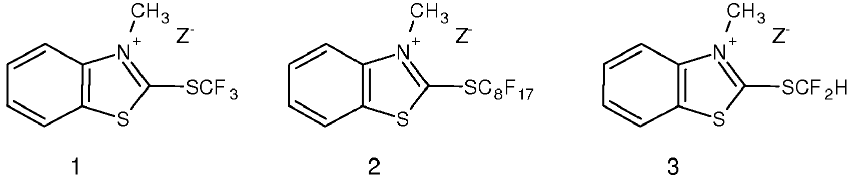 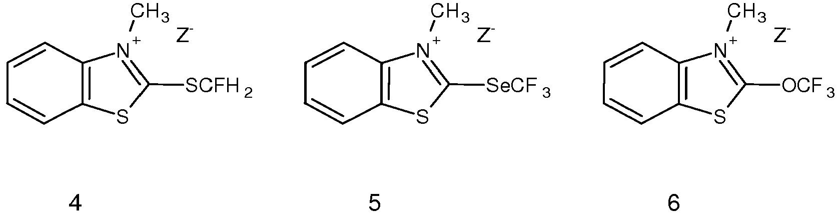 wherein Z⁻ is any of the above, preferably ⁻OTf.

It is to be understood that a methylsulfate salt of compound 3 is disclaimed.

The present compound is obtained in a process comprising the following steps:
- providing a compound of general formulae (V) 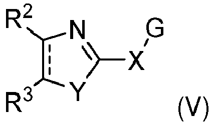 wherein G is H or a suitable leaving group as starting material;
- reacting the compound of general formulae (V) with at least one fluoroalkylating agent thereby providing an intermediate compound of general formulae (VI) 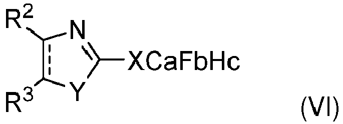
- alkylating the ring nitrogen of general formulae (VI) thereby providing the compound of general formulae (I) 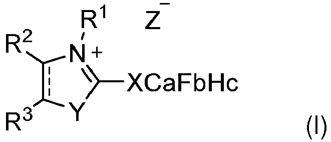 wherein R¹, R², R³, X, Y, Z, a, b, c have the above meanings.

A major advantage of the present compounds is their simple synthesis from relatively inexpensive precursor substrates.

As mentioned G may be H, in particular in case of electrophilic or oxidative fluoroalkylation, or a leaving group such as a halogen, -XR (such as -SR, -SeR), -CN or others, in particular in case for nucleophilic fluoroalkylation.

For example a suitable starting material would be R-SH, R-S-S-R or R-Se-Se-R wherein R may be a benzothiazole.

In an embodiment of the present process the fluoroalkylating agent is selected from a group containing a compound of general formulae (VII) NaSO₂CₐF_{b}H_{c} wherein a, b and c have the above meanings. A preferred variant of a fluoroalkylating agent is NaSO₂CF₃ (Langlois reagent). The reaction may be carried out under photoredox catalysis conditions.

The reaction of compound (V) with at least one fluoroalkylating agent of general formulae (VII) may be carried in the presence of an oxidizing agent such as K₂S₂O₈ or a photocatalyst, such as 9-mesityl-10-methylacridinium perchlorate or [Ir(dF(CF₃)ppy)(dtbbpy]PF₆ (dF(CF₃)ppy = 2-(2,4-difluorophenyl)-5-(trifluoromethyl)pyridine, dtbbpy = 4,4'-di-tert-butyl-2,2'-biyridine). In the latter case R-S-S-R or R-Se-Se-R may be used as starting materials and photoredox conditions such as blue light irradiation may be applied.

In another embodiment of the present process at least one fluoroalkylating agent is selected from a group containing a compound of general formulae (VIII) Hal CₐF_{b}H_{c} wherein Hal is I, Br, or Cl and wherein a, b, c have the above meanings. A preferred variant of a fluoroalkylating agent of formulae (VIII) is ICₐF_{b}H_{c}, such as IC₈F₁₇. The fluoroalkylating agent of formulae (VIII) is employed together with a suitable base (such as NaH) under heating or irradiation with UVA light.

In another embodiment of the present process at least one alkylating agent selected from a group containing alkyl trifluoromethanesulfonate, alkyl iodide, alkyl sulfate is employed for alkylating the intermediate compound of general formulae (VI).

As pointed out above, the present compound can be used as a nucleophilic reagent for transferring a fluorine-containing functional group onto high value organic compounds, in particular pharmaceutical or agrochemical targets.

The invention is explained in more detail by means of the following examples.

### Example 1: Synthesis of 3-methyl-2-((trifluoromethyl)thio)benzo[d]thiazol-3-ium trifluoromethanesulfonate) (Compound 1)

Compound **1** is prepared in two steps from the cheap starting material 2-mercaptobenzothiazole (MBT) which is a bulk chemical used in the industrial vulcanisation of rubber.

The trifluoromethyl group can be installed using the relatively inexpensive reagent NaSO₂CF₃ (Langlois' reagent) using published procedures leading to the intermediate compound. This species is a stable, non-reactive heteroaromatic compound that can be readily purified without decomposition.

In a second step, methylation of the ring nitrogen using methyl trifluoromethanesulfonate cleanly affords the reagent **1** which is obtained as a pure compound upon simple filtration. Although the Langlois reagent is used as the CF₃ source, the two-step synthetic strategy towards compound **1** can potentially be modified to enable even less expensive trifluoromethylthiolating reagents to be employed.

In particular, nucleophilic trifluoromethylation of disulfides or other sulfur compounds has been reported using the least expensive and therefore most industrially-attractive CF₃ source fluoroform (HCF₃).

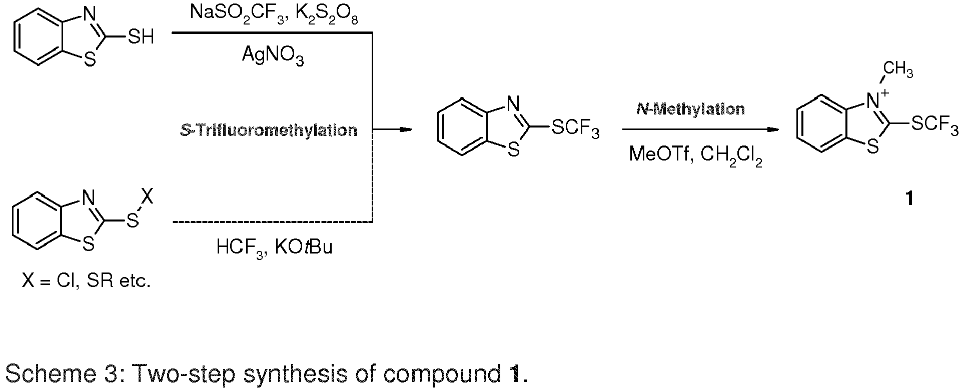

### Analytical data:

¹H NMR (400 MHz, Acetonitrile-*d*₃) δ = 8.40 (d, *J*=8.0, 1H, H_{Ar}), 8.28 (d, *J*=8.6, 1H, H_{Ar}), 8.05 (t, *J*=8.0, 1H, H_{Ar}), 7.97 (t, *J*=7.9, 1H, H_{Ar}), 4.45 (s, 3H, CH₃).
¹⁹F NMR (376 MHz, Acetonitrile-*d*₃) δ = -39.2 (SCF₃), -79.3 (S(O)₂CF₃).
¹³C NMR (151 MHz, Acetonitrile-*d*₃) δ = 160.6 (C_{q}), 143.8 (C_{q}), 134.1 (C_{q}), 132.4 (CH), 131.5 (CH), 127.7 (q, *J*=314, SCF₃), 125.2 (CH), 122.0 (q, *J*=321, S(O)₂CF₃), 119.4 (CH), 40.0 (CH₃).
HR-MS (ESI): m/z calculated for [C₉H₇F₃NS₂]⁺ ([M-(SO₂CF₃)]⁺): 249.9967, measured: 249.9955.
IR (ATR): v (cm⁻¹): 3086, 1664, 1578, 1490, 1476, 1462, 1433, 1382, 1279, 1266, 1240, 1227, 1196, 1173, 1161, 1150, 1108, 1092, 1050, 1026, 998, 814, 760, 722, 714, 688.

### Example 2

Compound 1 was employed in deoxytrifluoromethylthiolation reactions of aliphatic alcohols (see scheme 4a). This reaction is very useful as hydroxy groups are widespread in organic molecules and the ability to directly convert them into SCF₃ groups avoids the preparation of alkyl halide or pseudohalide precursors, reducing the overall number of synthetic steps required to prepare SCF₃-containing molecules.

Existing published methods for conducting this reaction use super-stoichiometric amounts of expensive AgSCF₃, CuSCF₃ or electrophilic trifluoromethylthiolating reagents in combination with excess Lewis acid or tetrabutylammonium iodide activators (Qing, Angew. Chem. Int. Ed. 2015; Rueping, Chem. Eur. J. 2014; Billard, Eur. J. Org. Chem. 2016; Magnier, Eur. J. Org. Chem. 2017). Most methods also require high reaction temperatures.

By contrast, the same process can be achieved using only a slight excess of metal-free compound **1** (1.25 equivalents) in acetonitrile (0.5 M) at room temperature with just 2 equivalents of an organic amine base (such as di(isopropyl)ethyl amine).

In addition to acting as a reagent for deoxytrifluoromethylthiolation, compound **1** can also serve as a more general source of ⁻SCF₃ upon addition of a suitable metal or ammonium salt activator. This could potentially be achieved catalytically; AgSCF₃, for example, could be generated *in situ* upon activation of **1** with small amounts a soluble, inexpensive silver(I) salt such as Ag₂O or AgNO₃ (see Scheme 4b).

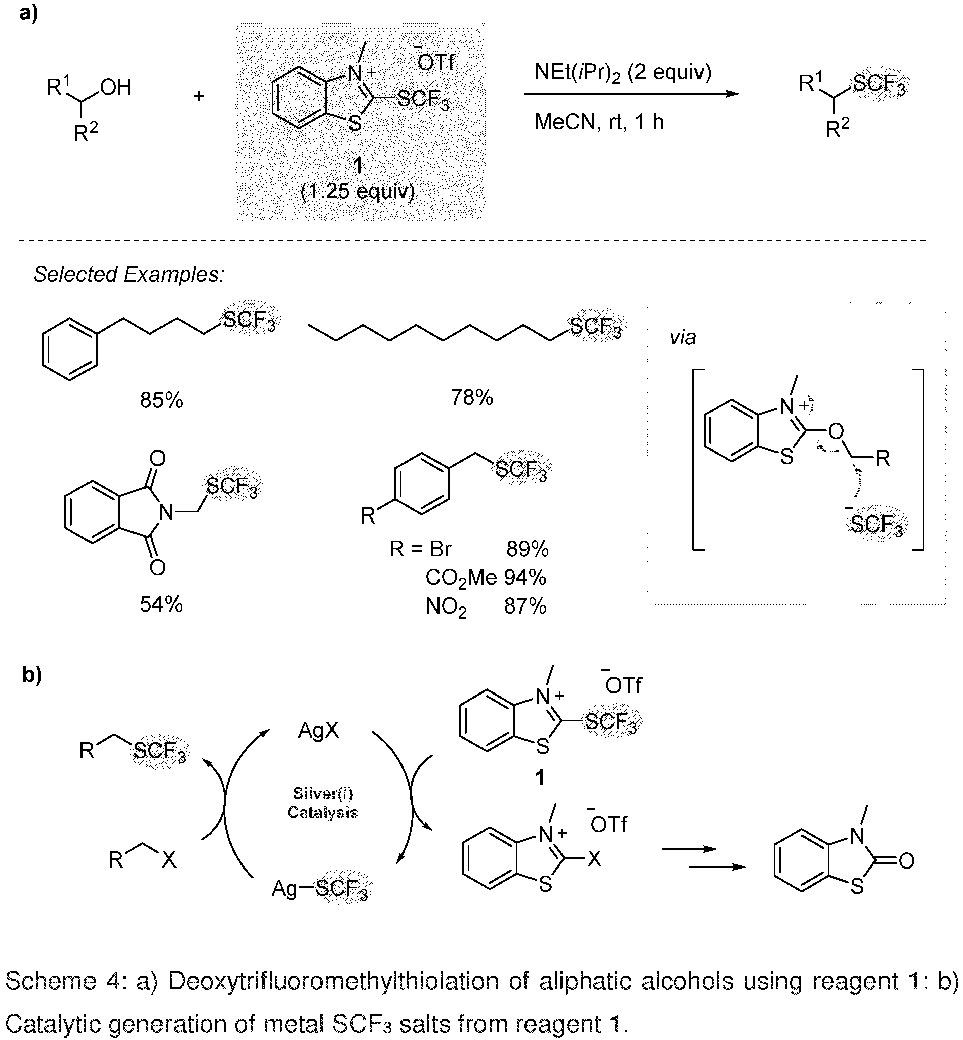

### Example 3: Synthesis of 3-methyl-2-((perfluorooctyl)thio)benzo[d]thiazol-3-ium trifluoromethanesulfonate (compound 2)

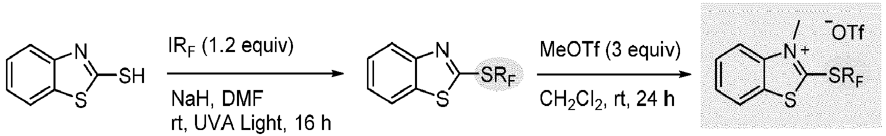

3-Methyl-2-((perfluorooctyl)thio)benzo[d]thiazol-3-ium trifluoromethanesulfonate was synthesized in two steps from 1-mercaptobenzothiazole. In the first step the C₈F₁₇ group was installed using a modified literature procedure employing the commercially-available perfluoroalkyl iodide IC₈F₁₇ and sodium hydride under irradiation with UVA light. N-Methylation using methyl trifluoromethanesulfonate was then conducted in analogy to the method used in Example 1.

### Analytical data:

¹H NMR (400 MHz, Acetonitrile-*d*₃) δ = 8.40 (d, *J*=8.4, 1H, H_{Ar}), 8.29 (d, *J*=8.7, 1H, H_{Ar}), 8.07 (t, *J*=7.6, 1H, H_{Ar}), 7.99 (t, *J*=7.8, 1H, H_{Ar}), 4.47 (s, 3H, CH₃).
¹⁹F NMR (376 MHz, Acetonitrile-*d*₃) δ = -79.3 (S(O)₂CF*₃*), -81.4 (t, *J*=10*,* CF₃), -82.5 (t, *J*=14, SCF₂), -118.4 - -118.6 (m, CF₂), -121.2 - -121.5 (m, CF₂), -121.8 - -122.1 (m, CF₂), -122.1 - -122.3 (m, CF₂), -122.9 - -123.1 (m, CF₂), -126.4 - -126.6 (m, CF₂). HR-MS (ESI): m/z calculated for [C₁₆H₇F₁₇NS₂]⁺ ([M]⁺): 599.9743, measured: 599.9730.
IR (ATR): v (cm⁻¹): 3101, 2361, 1576, 1490, 1461, 1435, 1370, 1328, 1281, 1251, 1200, 1149, 1134, 1097, 1054, 1031, 958, 930, 849, 816, 798, 768, 756, 741, 723, 714, 707, 655.

### Example 4: Synthesis of 3-methyl-2-((trifluoromethyl)selanyl)benzo[d]thiazol-3-ium trifluoromethanesulfonate (compound 5)

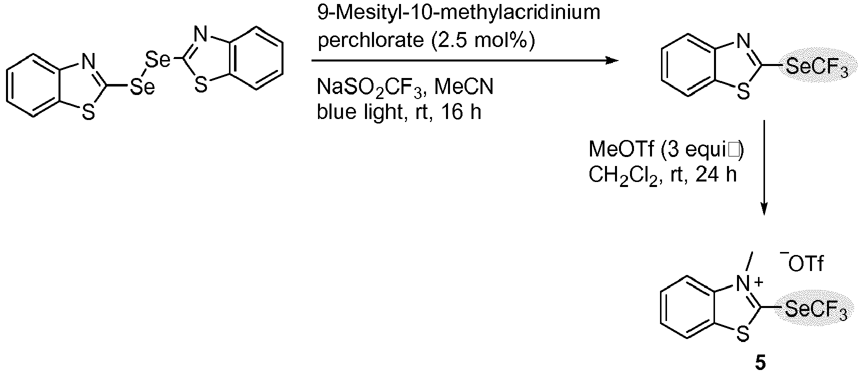

3-Methyl-2-((trifluoromethyl)selanyl)benzo[d]thiazol-3-ium trifluoromethanesulfonate was synthesized in two steps from 1,2-bis(benzo[d]thiazol-2-yl)diselane. In the first step the CF₃ group was installed under photoredox catalysis conditions using NaSO₂CF₃. N-Methylation using methyl trifluoromethanesulfonate was then conducted in analogy to the method used in Example 1.

The reactivity exhibited by the selenium derivative (compound **5**) is similar to the reactivity of compound **1**.

### Analytical data:

¹H NMR (400 MHz, Acetonitrile-*d*₃) δ = 8.37 (d, *J*=7.8, 1H, H_{Ar}), 8.27 (d, *J*=8.6, 1H, H_{Ar}), 8.01 (t, *J*=8.3, 7.9, 1H, H_{Ar}), 7.93 (t, *J*=7.9, 1H, H_{Ar}), 4.47 (s, 3H, CH₃).
¹⁹F NMR (376 MHz, Acetonitrile-*d₃*) δ = -32.7 (SeCF₃), -79.3 (S(O)₂CF₃).
¹³C NMR (126 MHz, Acetonitrile-*d₃*) δ = 159.2 (C_{q}), 143.9 (C_{q}), 135.0 (C_{q}), 132.1 (CH), 131.1 (CH), 125.1 (CH), 122.7 (q, *J*=337, SeCF₃), 122.0 (q, *J*=321, S(O)₂CF₃), 119.3 (CH), 41.4 (CH₃).
CHNS Elemental Analysis: calculated for C₁₀H₇F₆NO₃S₂Se: C 26.92; H 1.58; N 3.14, S 14.37; measured: C 27.00, H 1.97, N 3.14, S 14.50.
IR (ATR): v (cm-1): 3098, 3064, 1577, 1489, 1461, 1442, 1388, 1252, 1223, 1187, 1151, 1140, 1101, 1079, 1054, 1043, 1028, 987, 962, 802, 766, 741, 729, 712.

## Claims

1. A fluorine containing compound according to the general formulae (I) 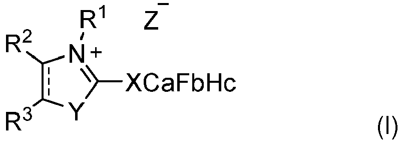 wherein
- R¹ is C1-C20 alkyl;
- R², R³ are in each case an alkyl, a cycloalkyl, an aryl, a heteroaryl, halogen, fluoroalkyl or both R² and R³ are part of a cyclic system;
- X is S, O, Se, Te; preferably S, O, Se;
- Y is S, O, NR⁵ where R⁵ is an alkyl or aryl;
- Z⁻ is R⁴SO₃⁻ with R⁴ being H, C1-C10 alkyl, aryl, CₐF_{b}H_{c}, in particular - OTf (CF₃SO₃⁻), p-Tos; Ph-SO₃-; I⁻, Cl⁻, ClO₄⁻, BF₄⁻;
- a is 1-20, preferably 1-12, more preferably 1-8;
- b is (2a+1) - c;
- c is 0-10, preferably 0-5, more preferably 0, 1, 2.

2. Compound according to claim 1, **characterized in that** R¹ is C1-C10 alkyl, preferably C1-C5 alkyl, more preferably C1-C3 alkyl.

3. Compound according to claim 1 or 2, **characterized in that** R² and R³ are part of an aromatic system, preferably of a C6 aryl ring.

4. Compound according to one of the preceding claims, **characterized in that** the moiety R⁵ is a C1-C10 alkyl, preferably C1-C5 alkyl, more preferably C1-C3 alkyl, or a C6-C10 aryl, preferably a C6 aryl.

5. Compound according to one of the preceding claims, **characterized by** the general formulae (II) 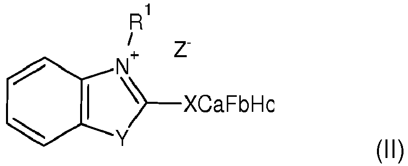 wherein R¹, X, Y, Z, a, b, c have the above meanings.

6. Compound according to one of the preceding claims, **characterized by** the general formulae (III) 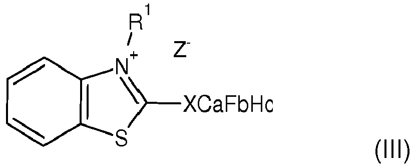 wherein R¹, X, Z, a, b, c have the above meanings.

7. Compound according to one of the preceding claims, **characterized by** the general formulae (IV) 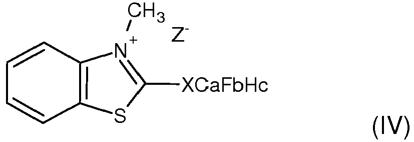 wherein X, Z, a, b, c have the above meanings.

8. Process for obtaining a compound according to one of the preceding claims, comprising the following steps:
- providing a compound of general formulae (V) 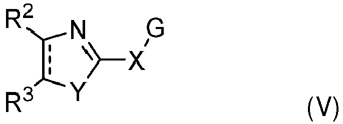 wherein G is H or a leaving group as starting material;
- reacting the compound of general formulae (V) with at least one fluoroalkylating agent thereby providing an intermediate compound of general formulae (VI) 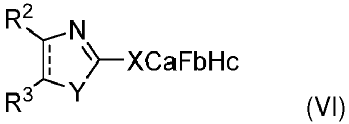
- alkylation of the ring nitrogen of general formulae (VI) thereby providing the compound of general formulae (I) 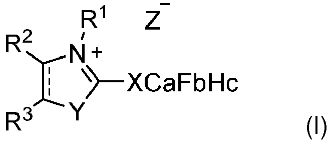 wherein R¹, R², R³, X, Y, Z, a, b, c have the above meanings.

9. Process according to claim 8, **characterized in that** at least one fluoroalkylating agent is selected from a group containing a compound of general formulae (VII) NaSO₂CₐF_{b}H_{c} wherein a, b, c have the above meanings.

10. Process according to claim 8, **characterized in that** at least one fluoroalkylating agent is selected from a group containing a compound of general formulae (VIII) Hal CₐF_{b}H_{c} wherein Hal is I, Br, or Cl and wherein a, b, c have the above meanings.

11. Process according to claim 8-10, **characterized in that** at least one alkylating agent selected from a group containing alkyl trifluoromethanesulfonate, alkyl iodide, alkyl sulfate is employed.

12. Use of a compound according to one of claims 1 - 7 as a nucleophilic reagent for transferring a fluorine-containing functional group onto high value organic compounds, in particular pharmaceutical and agrochemical targets.
